## Europäisches Patentamt
## European Patent Office
## Office européen des brevets

(11) Publication number: **0 293 184**
A2

(12)
# EUROPEAN PATENT APPLICATION

(21) Application number: 88304735.9

(22) Date of filing: 25.05.88

(51) Int. Cl.⁴: **G 01 N 33/543**
G 01 N 33/569, C 12 N 15/00

(30) Priority: 29.05.87 US 56553

(43) Date of publication of application:
30.11.88 Bulletin 88/48

(84) Designated Contracting States:
AT BE CH DE ES FR GB GR IT LI LU NL SE

(71) Applicant: SMITHKLINE BECKMAN CORPORATION
One Franklin Plaza P O Box 7929
Philadelphia Pennsylvania 19103 (US)

(72) Inventor: Debouck, Christine Marie
667 Pugh Road
Wayne Pennsylvania 19087 (US)

Rosenberg, Martin
241 Mingo Road
Royersford Pennsylvania 19468 (US)

(74) Representative: Giddings, Peter John, Dr. et al
Smith Kline & French Laboratories Ltd. Corporate
Patents Mundells
Welwyn Garden City Hertfordshire AL7 1EY (GB)

Claims for the following Contracting States: ES + GR.

(54) **Immunodiagnostic assays using chimeric antigens.**

(57) An immunodiagnostic device comprising an antibody bound to a solid support and chimeric antigen having a first and second antigenic region the first antigenic region being reactive with the bound antibody but not being cross reactive with antibodies recognising said second antigenic region. In particular the device is used in the detection of specific disease states e.g. AIDS or hepatitis B.

EP 0 293 184 A2

0 293 184

Description

# IMMUNODIAGNOSTIC ASSAYS USING CHIMERIC ANTIGENS

Field of the Invention

This invention relates to chimeric antigens for use in an assay for diagnosis and prognosis of infection or other disease states.

Background of the Invention

Immunodiagnostic assays are based on the binding affinity between an antigen and its corresponding antibody. When an antigen-antibody complex is formed, the complex can be detected using a variety of detection systems, for example, radioactive isotopes, fluorescent tags, or enzymes such as horseradish peroxidase.

Use of immunoassay techniques have been standard laboratory practice for many years. The basic concept in all of the various assays has been the binding of a labeled antigen to a preselected antibody and thereafter measuring the amount of bound complex. More recently, use of a monoclonal antibody rather than a polyclonal antibody has resulted in increased sensitivity and specificity of the assay. However, the use of monoclonal rather than polyclonal antibodies does not completely eliminate the problem of specificity or degree of sensitivity of the particular assay. Depending upon the antibody-antigen complex being formed there still remains competition for particular binding sites on the molecule.

Many immunoassays depend upon the binding complex recognizing the same antibody as used for initial binding to the solid support. Reliance on the same antibody at two critical points in the assay can result in reduced sensitivity again due to competition for available binding sites. In addition, lack of specificity of the antibody can result in the generation of "false positives" especially in diagnostic assays where stages of disease progression are being diagnosed and recognition of epitopes associated with a particular disease stage is important. Attempts to eliminate this problem have resulted in the "two-site" or "sandwich" immunoassay. With this type of immunoassay, the antigen of interest has two antibodies bound to its surface, one of which is radiolabeled or has bound to it some other detection molecule and the other of which is bound to a solid support. Thus, the competition for particular binding sites on the molecule is reduced. The sandwich assay can be further enhanced by the use of highly purified antibodies.

An example of a disease for which early diagnosis as well as identification of the stage of the disease can be important is acquired immunodeficiency syndrome (AIDS). HIV, the causative agent of AIDS and related disorders, is a member of the Retroviridae family. There exist several isolates of HIV including human T-lymphotropic virus type-III (HTLV-III), the lymphadenopathy virus (LAV) and the AIDS-associated retrovirus (ARV). A related retrovirus, designated HIV type 2, was shown recently to be associated with AIDS in West Africa. Guyader, et al., Nature 326:662(1987).

Molecular characterization of the HIV genome has demonstrated that the virus exhibits the same overall gag-pol-env organization as other retroviruses. Ratner, et al., Nature 313:277 (1985), Wain-Hobson, et al., Cell 40:9(1985). In addition, it contains at least five genes that are not found in more ordinary retroviruses: sor, tat3, art/trs, 3'orf and R.

The severity of AIDS makes early and accurate diagnosis of infection by HIV and detection and elimination of HIV-contaminated samples from blood banks extremely important. Gallo, et al., U.S. Patent 4,520,113, disclose use of antigens derived from HTLV-III to detect presence of anti-HTLV-III antibodies in serum. Montagnier, et al., EP-A-138,667, disclose use of a specified LAV antigen to detect infection by the virus. Papas, et al., United States Patent Application Serial No. 6-664,972 (Derwent Accession No. 85-110268/18), disclose expression of a HTLVv-I envelope protein coding sequence in E. coli and use of the protein expressed thereby to detect infection by HTLV-I. Crowl, et al., Cell 41:979(1985), report expression in E. coli of portions of the HTLV-III envelope protein gene, env, and use of such proteins for detection of infection by HTLV-III. Casey, et al., J. Virol. 55:417(1985), report purification of one gag gene product, an internal structural protein of HTLV-III referred to as p24, and use of the protein to detect infection by HTLV-III.

Many researchers in the field have been working on the expression in bacteria of the various antigens of the AIDS retrovirus. Recently, novel peptides with sequences of particular fragments of HIV have been constructed for use as reagents for detection of exposure to the AIDS virus. Cosand, U.S. Patent 4,629,783; Capon, et al., EP-A-187041. Use of a recombinant polypeptide from the endonuclease region of the AIDS retrovirus polymerase gene (pol) to detect serum antibodies in infected individuals is described by Steimer, et al., J. Virology 58:9(1986).

A polypeptide expressed by cells transformed with a recombinant vector containing HTLV-III cDNA encoding the env gene sequence and the use of such immunoreactive polypeptide in assays for the detection of HTLV-III is described in Chang, EP-A-185,444. Dowbenko, et al. (P.N.A.S. 82, 774-8, 1985.) disclose the expression in E. coli of the AIDS retrovirus p24 gag protein and its use as a diagnostic reagent. Synthesis in E.

coli of the HTLV-III core antigens and immunoreactivity with human serum is also reported by Ghrayeb, et al., DNA 5:93(1986) and Steimer, et al., Virology 150:(1986).

## Summary of the Invention

In one embodiment of the invention an immunodiagnostic device for assaying for the presence of antibodies directed against a particular antigen is described. The device comprises a solid support, an antibody bound to the solid support and a chimeric antigen having a first and second antigenic region, the first antigenic region being reactive with the bound antibody but not being cross-reactive with antibodies recognizing the second antigenic region.

Another embodiment of the invention is a process for preparing chimeric HIV-antigens which comprises fusing a portion of the E. coli galactokinase (galK) gene to an HIV gene segment and introducing the resulting fused gene segment into an organism and culturing the organism such that the chimeric HIV-antigen is expressed.

In another embodiment of the invention, an expression vector comprising a promoter and a portion of the galK gene flanked by a coding sequence for an antigen not cross-reactive with galK is described.

In still another embodiment of the invention, a method for detection of anti-HIV antibodies in a sample is set forth. Detection of the antibodies is accomplished by contacting the sample with a galK-HIV chimeric antigen bound to a solid support such that only those samples containing HIV specific immunoglobulins bind to the chimeric HIV-antigen.

## Detailed Description of the Invention

The chimeric antigen of the subject invention is a fusion protein containing a portion of an E. coli gene encoding a gene product incapable of cross-reaction with the protein of interest fused to a gene segment encoding the protein of interest. The gene segment of interest can be one which encodes a protein associated with particular diseases or disease states such as sexually transmitted diseases, e.g., AIDS, gonorrhea, syphilis, chlamydia or hepatitis B. Other specific gene products refelective of a particular disease or disease state such as proteins associated with specific cancers, genetic abnormalities, other bacterial, fungal, viral or parasitic diseases, e.g., Plasmodium, Trypanosoma, Candida, Pichia, Salmonella, Pseudomonas and the like may also be expressed as fusion proteins.

Because a portion of the chimeric antigen is specifically directed to a particular disease or disease state, use of the chimeric antigen for diagnosis as well as prognosis of a disease is advantageous. The use of a fusion system, for example, the E. coli galK system, also provides a means to obtain high level expression of the chimeric antigen especially when the chimeric antigen is poorly or not expressed from its unfused gene segment. The galK portion of the chimeric antigen acts as a "handle" to which other specific proteins can be attached. Because of its specificity the chimeric antigen also provides an effective means of protein purification. Other galK-like "handles" which may be used include portions of the influenza NS-1 protein, the phage Lambda 0 protein, the E. coli β-galactosidase protein, or any protein which is well expressed in E. coli or the recombinant host of interest, e.g., other bacteria, yeast, higher eucaryotic cells. However, selection of the handle must be such that the handle portion of the fusion protein is capable of immunologic recognition yet is not recognized by binding sites in the sample.

The chimeric antigens are useful in an immunodiagnostic device which provides high specifity for the protein of interest along with increased sensitivity of the assay. Because a portion of the chimeric antigen is not recognized by the sample, e.g., human or animal blood, plasma, serum, saliva, sputum, excreta or other body fluids, all epitopes of the protein of interest are available for binding to the sample. This feature greatly enhances the specificity and is an improvement over use of specific monoclonal antibodies. With monoclonal antibodies it is possible for two monoclonal antibodies to recognize overlapping epitopes on the antigen of interest and therefore compete for binding sites.

As a result of the unique specificity of the galK handle, the use of the chimeric antigen for an immunodiagnostic assay results in a rapid, highly specific and sensitive assay capable of detecting a particular disease and also in many instances a particular stage of the disease. This is particularly true when used for HIV antibody detection as recent evidence has suggested a correlation between the presence or absence of antibodies to specific HIV antigens and disease progression.

The immunodiagnostic system of the subject invention is a capture immunoassay in which an antibody bound to the solid support has binding sites specific for sites on one portion of the chimeric antigen. The chimeric antigen is capable of binding to the antibody bound to the solid support while still maintaining free for binding all epitopes of the antigenic portion derived from the causative agent of a particular disease or disease state. The site necessary for binding to the solid support is distinct from the site used to bind the sample. In particular, the chimeric antigen is so constructed that the galK site or other fusion partner used to make the chimeric antigen is not recognized by the sample being tested. Therefore, all available sites on the chimeric antigen which are derived from the causative agent for the disease or disease state are available for binding.

One embodiment of this invention is the use of the capture immunodiagnostic system to detect antibodies to the individual viral proteins and virally induced proteins associated with HIV infection. A galK-specific antibody (polyclonal or monoclonal) is used in a capture immunodetection assay, i.e., enzyme linked immunosorbent assay (ELISA), "sandwich immunoassay", competitive binding assay or radioimmune assay (RIA) for the detection of HIV seroconversion. The design of the capture assay maximizes the binding of patient sample to specific chimeric HIV-antigens associated with particular stages of the disease thereby providing a quick and highly sensitive diagnostic test.

In such a capture assay, the galK-specific antibody is first attached to a solid support (e.g., polystyrene 96-well plates or beads) and the impurities are washed away. Then, a chimeric galK-HIV antigen in either purified or semi-purified form is allowed to interact with the galK antibody. All unbound impurities are washed away. Finally, the sample, for example, human serum or other body fluids, is allowed to react with the chimeric HIV-antigen bound via galK antibody to the support and all unbound material is washed off. The human immunoglobulins reactive with the bound chimeric HIV-antigen can be detected by a variety of procedures, e.g., fluorescence spectrophotometry, colorimetry using enzymes such as horseradish peroxidase, radiolabeled isotope tagging with isotopes such as $^{14}C$ or $^{125}I$, or other detection systems known in the art.

The advantages of the capture system described above are three-fold: (1) it is not necessary to purify the galK-HIV antigen chimera to homogeneity as the galK-specific antibody will attach to the amino-terminus of the chimeric antigen specifically and maintain it anchored on the solid support through the washes that eliminate all impurities present in the chimeric protein preparation; (2) as the chimeric protein is attached on the solid support via its galK "handle", all HIV epitopes present on the molecule are available for recognition by the patient sample. This acts to increase the sensitivity of the assay as compared to the classic capture ELISA assay in which the monoclonal antibodies used for the capture and the detection are both directed against the antigen of interest; and (3) specificity is increased as the antibody in the sample will only recognize the chimeric antigen if it is specifically directed against that particular antigen.

The HIV antigens of interest can be expressed as translational fusions to the amino-terminal region of the protein encoded by the E. coli galK gene. The E. coli expression system was chosen in order to maximize the role of the galK gene in increasing the level of expression of gene segments, especially if the gene segments are poorly or not expressed when unfused. However, other expression systems, e.g. yeast (S. cerevisiae), Streptomyces, insect cells such as Drosophila and higher eucaryotic cells (mammalian cells), may also be used.

Examples of expression systems which can be used to express the chimeric antigens of the invention in E. coli are, for example, influenza NS1, Young et al., Proc. Natl. Acad. Sci. USA 80:6105(1983), phage lambda O protein, Rosenberg, et al., Meth. Enzymol. 101:123(1983), and the E. coli β galactosidase protein, Bartus, et al., Antim. Ag. Ch. 25:622(1984). Expression in Streptomyces includes use of E. coli galK, Brawner, et al., Gene 40:191(1985), the genes from the Streptomyces gal operon, Fornwald, et al., Proc. Natl. Acad. Sci. USA 84:2130(1987), and the Streptomyces β galactosidase, Burnett, et al., in Microbiology 1985, ed. Levin, Amer. Soc. Microbiol., Washington, D.C. (1985). In yeast, expression can be achieved with E. coli galK, Rosenberg, et al., in Genetic Engineering, vol. 8, ed. Setlow, Plenum Press (1986), and 3-phosphoglycerate kinase (PGK), Chen, et al., Nucleic Acids Res. 12:8951(1984). In mammalian cells, Schumperli, et al., Proc. Natl. Acad. Sci. USA 79:257(1982) has expressed E. coli galK; such system may also be useful as a translational fusion expression system.

The bacterium E. coli is the organism of choice for the production of the chimeric HIV-antigens for diagnostic purposes. Indeed, cloning and expression can be obtained rapidly in E. coli and to date in some cases, much higher levels of gene expression have been achieved in E. coli as compared to other organisms, e.g., mammalian systems (Chinese hamster ovary cells) or yeast. In addition, production in E. coli is readily amenable to large-scale fermentation and protein purification.

Use of the galK fusion system allows large quantities of specific HIV-antigens to be generated. The specific chimeric HIV-antigens so expressed can be used for the detection of HIV-seroconversion in any of the recognized diagnostic immunoassays, e.g., Western blot, dot/slot blot, direct ELISA or other such immunodetection assays.

The E. coli galK translational fusion vector, plasmid pOTSKF33 was constructed as specifically described in Example 1. Other fusion vectors can be prepared using the techniques described in the examples.

The pOTSKF33 fusion vector has been used for overexpression of several foreign gene products in E. coli as chimeric proteins such as human beta-globin, tissue plasminogen activator, glucocorticoid receptor, and metallothionein. These proteins are poorly expressed when unfused. It was, therefore, found to be useful for the overexpression of the various gene products from HIV.

The genes from HIV on which initial experiments were focused included gag, pol and env as these encode the proteins that are most recognized by patient sera. The HIV gag region is initially translated into a polyprotein precursor of approximately 55 kilodaltons (kD) which is then processed into the mature p17, p24 and p15 gag structural proteins.

The gag-pol region is believed to be translated into a large precursor via a translational frameshift between the overlapping gag and pol reading frames. Ratner, et al., Nature 313:277(1985). The gag-pol precursor is post-translationally processed to yield the mature gag proteins and the products of the pol region including the reverse transcriptase and endonuclease.

The env region is translated as a gp160 precursor polypeptide that is processed at two or more sites. The first processing event removes a secretion signal peptide of approximately 30 amino acids and the second processing event yields the outermembrane gp120 region and gp41 which consists of a hydrophobic region that spans the membrane followed by a hydrophilic cytoplasmic anchor.

The pOTSKF33 fusion vector has the strong and regulatable $P_L$ promoter flanked by a 56 codon-long portion of the galK gene immediately followed by restriction sites for gene fusion, e.g., to the HIV gene segments of interest. It has been found that a 253 codon-long portion of galK when inserted into an expression plasmid (fusion vector pOTSKF66) can also be fused to the genes of interest, and results in high level expression.

The full-length galK gene product accumulates to high levels and is stable when expressed in E. coli using the pAS1 system (Rosenberg, et al., supra). In addition, fusion of heterologous genes to the amino-terminal end of the galK gene product was found to result in stable, i.e., not degraded or otherwise altered or inactivated fusion product. High level expression, i.e., 10% or greater of total cellular protein of the HIV-derived antigens, can be obtained and is desirable in order to facilitate the large-scale production of these antigens through bulk-scale fermentation and various purification processes.

The specific HIV antigens produced by E. coli can be used to stimulate production of antiserum which is reactive with specific sites on HIV proteins. Thus, each chimeric antigen constructed and expressed can be used as an agent for the production of site-directed antibodies. The ability to raise polyclonal antibodies renders it possible to also produce monoclonal antibodies by the techniques as originally described by Kohler and Milstein, Nature 256:495(1975) or other techniques of cell fusion or transformation. The polyclonal or monoclonal antibodies so produced are useful in the capture ELISA, as described in Western blot analysis, dot/slot blot analysis, or may also be used for affinity purification. The antibodies are also useful in direct immunodetection of HIV antigens (free antigen, free virus, infected cells) in patient samples.

## Examples

The examples which follow are illustrative, and not limiting of the invention.

## EXAMPLE 1

Construction of the galK translation fusion vector, pOTSKF33:

pOTSKFF33 derives from the pAS1 expression vector (Rosenberg, et al., supra) and comprises a portion of the E. coli galK gene (56 codons) flanked by a polylinker with restriction sites for fusion in any of the three translation frames, stop codons for each phase and some additional cloning sites. It was constructed in several steps:

Step 1: Insertion of the polylinker region of pUC19 into the pOTS34 vector:

pOTS34, a derivative of pAS1 (Devare, et al., Cell 36:43(1984), was digested with the BglII restriction endonuclease, treated with DNA polymerase I (Klenow) to create blunt ends and redigested with the SphI restriction endonuclease. The large 5251 base pair (bp) BglII-SphI fragment was purified and ligated to the 135 bp PvuII-SphI restriction fragment isolated and purified from pUC19 (Yanisch-Perron, et al., Gene 33:103(1985). The resulting plasmid was called pOTS-UC.

Step 2: Insertion of the partial galK expression unit from pASK into pOTS-UC:

pOTS-UC was digested with XmaI, treated with Klenow to create blunt ends and redigested with HindIII. The large fragment was purified and ligated to a 2007 bp BclI filled-HindIII fragment isolated and purified from pASK. The resulting plasmid was called pOTSKF45.

The pASK plasmid vector is a pAS1 derivative in which the entrie galK coding sequence is inserted after the ATG initiation codon. This galK sequence differs from the authentic sequence (Debouck, et al., Nucleic Acids Res. 13:1841(1985)) at the 5' end:

  - pASK sequence: ATG.GAT.CCG.GAA.TTC.CAA.GAA.AAA...
  - authentic seq: ATG.AGT.CTG.AAA.GAA.AAA...

The pASK sequence contains a BamHI site (GGATCC) at the ATG whereas the authentic sequence does not.

Step 3: Introduction of a linker with translation stops into pOTSKF45:

pOTSKF45 was digested with XmaI and SalI and then treated with Klenow to create blunt ends. The linker 5'-GTA.GGC.CTA.GTT.AAC.TAG -3' was then introduced between the XmaI filled and SalI filled sites by ligation with T4 DNA ligase to yield the final vector: pOTSKF33.

## EXAMPLE 2

Construction of galK-HIV protein fusions:

Construction of galK-p24 gag fusion:
A 567 bp PvuII-HindIII filled-in fragment containing the last 13 codons of p17 gag and codons number 1-177 of p24 gag, was inserted at the XmaI filled-in site of pOTSKF33 for in-frame fusion to the first 56 codons of galK. The endpoints of this fragment were PvuII (689) and HindIII (1256) using the numbering of Ratner, et al., Nature 313:277(1985). The fragment was isolated from the BH10 clone of the HTLV-III(b) isolate (Hahn, et al., Nature 312:166 (1984)) using standard cloning techniques. The amino acid sequence, using the one letter code for amino acid designation, of the galK-p24 gag fusion follows:

Length: 253 amino acid residues

```
  1   MDPEFQEKTQ  SLFANAFGYP  ATHTIQAPGR  VNLIGEHTDY  NDGFVLPCAI
 51   DYQTVIPADT  GHSSQVSQNY  PIVQNIQGQM  VHQAISPRTL  NAWVKVVEEK
101   AFSPEVIPMF  SALSEGATPQ  DLNTMLNTVG  GHQAAMQMLK  ETINEEAAEW
151   DRVHPVHAGP  IAPGQMREPR  GSDIAGTTST  LQEQIGWMTN  NPPIPVGEIY.
201   KRWIILGLNK  IVRMYSPTSI  LDIRQGPKEP  FRDYVDRFYK  TLRAEQAVGL
251   VN*
```

Construction of galK-p55 gag fusion:
A 1266 bp ClaI-BglII filled-in fragment containing p17 gag (minus its first 14 codons), the complete p24 gag and the first 60 codons of p15 gag, was inserted at the StuI site of pOTSKF33 for in-frame fusion to the first 56 codons of galK. The Ratner coordinates for this fragment were: ClaI (374) and BglII (1640). The amino acid sequence of the galK-p55 gag fusion follows:

Length: 484 amino acid residues

```
  1   MDPEFQEKTQ  SLFANAFGYP  ATHTIQAPGR  VNLIGEHTDY  NDGFVLPCAI
 51   DYQTVIPGRR  WEKIRLRPGG  KKKYKLKHIV  WASRELERFA  VNPGLLETSE
101   GCRQILGQLQ  PSLQTGSEEL  RSLYNTVATL  YCVHQRIEIK  DTKEALDKIE
151   EEQNKSKKKA  QQAAADTGHS  SQVSQNYPIV  QNIQGQMVHQ  AISPRTLNAW
201   VKVVEEKAFS  PEVIPMFSAL  SEGATPQDLN  TMLNTVGGHQ  AAMQMLKETI
251   NEEAAEWDRV  HPVHAGPIAP  GQMREPRGSD  IAGTTSTLQE  QIGWMTNNPP
301   IPVGEIYKRW  IILGLNKIVR  MYSPTSILDI  RQGPKEPFRD  YVDRFYKTLR
351   AEQASQEVKN  WMTETLLVQN  ANPDCKTILK  ALGPAATLEE  MMTACQGVGG
401   PGHKARVLAE  AMSQVTNTAT  IMMQRGNFRN  QRKMVKCFNC  GKEGHTARNC
451   RAPRKKGCWK  CGKEGHQMKD  CTERQANFLG  KIL*
```

Construction of galK-RTendo fusion:
A 2129 bp HincII-EcoRI filled-in fragment containing the last 9 codons of the protease, the complete reverse transcriptase and the first half, codons number 1-141, of endonuclease was inserted at the SmaI site of pOTSKF33 for in-frame fusion to the first 56 codons of galK. The Ratner coordinates for this fragment were: HincII (2099) and EcoRI (4228).
The amino acid sequence of the galK-RTendo fusion follows:
Length: 769 amino acid residues

```
  1    MDPEFQEKTQ SLFANAFGYP ATHTIQAPGR VNLIGEHTDY NDGFVLPCAI
 51    DYQTVIPTQI GCTLNFPISP IETVPVKLKP GMDGPKVKQW PLTEEKIKAL
101    VEICTEMEKE GKISKIGPEN PYNTPVFAIK KKDSTKWRKL VDFRELNKRT
151    QDFWEVQLGI PHPAGLKKKK SVTVLDVGDA YFSVPLDEDF RKYTAFTIPS
201    INNETPGIRY QYNVLPQGWK GSPAIFQSSM TKILEPFKKQ NPDIVIYQYM
251    DDLYVGSDLE IGQHRTKIEE LRQHLLRWGL TTPDKKHQKE PPFLWMGYEL
301    HPDKWTVQPI VLPEKDSWTV NDIQKLVGKL NWASQIYPGI KVRQLCKLLR
351    GTKALTEVIP LTEEAELELA ENREILKEPV HGVYYDPSKD LIAEIQKQGQ
401    GQWTYQIYQE PFKNLKTGKY ARMRGAHTND VKQLTEAVQK ITTESIVIWG
451    KTPKFKLPIQ KETWETWWTE YWQATWIPEW EFVNTPPLVK LWYQLEKEPI
501    VGAETFYVDG AANRETKLGK AGYVTNKGRQ KVVPLTNTTN QKTELQAIYL
551    ALQDSGLEVN IVTDSQYALG IIQAQPDKSE SELVNQIIEQ LIKKEKVYLA
601    WVPAHKGIGG NEQVDKLVSA GIRKILFLDG IDKAQDEHEK YHSNWRAMAS
651    DFNLPPVVAK EIVASCDKCQ LKGEAMHGQV DCSPGIWQLD CTHLEGKVIL
701    VAVHVASGYI EAEVIPAETG QETAYFLLKL AGRWPVKTIH TDNGSNFTSA
751    TVKAACWWAG IKQEFGIG*
```

## Construction of galK-p41 env fusion:

A 301 Rsal-HindIII filled-in fragment containing codons number 29-129 of p41 env, was inserted at the Smal site of pOTSFK33 for in-frame fusion to the first 56 codons of galK. The Ratner coordinates for this fragment were: Rsal (7417) and HindIII (7718).

The amino acid sequence of the galK-p41 env fusion follows:

Length: 160 amino acid residues

```
  1    MDPEFQEKTQ SLFANAFGYP ATHTIQAPGR VNLIGEHTDY NDGFVLPCAI
 51    DYQTVIPQAR QLLSGIVQQQ NNLLRAIEAQ QHLLQLTVWG IKQLQARILA
101    VERYLKDQQL LGIWGCSGKL ICTTAVPWNA SWSNKSLEQI WNNMTWMEWD
151    REINNYTSWV
```

## Construction of galK-p120 env(N) fusion:

A 1790 bp Asp7181-HindIII filled-in fragment, containing a large portion of p160 env, was inserted at the Stul site of pOTSKF33 for in-frame fusion to the first 56 codons of galK. The resulting plasmid was digested with Stul and ligated to a linker containing translation stops in all three reading frames (5′ CTA.GTT.AAC.TAG 3′). This construct expresses the amino-terminal half (N) of p120 env from codons number 12-174. The Ratner coordinates for this segment are: Asp718I (5928) and Stul (6411).

The amino acid sequence of the galK-p120 env(N) fusion follows:

Length: 224 amino acid residues

```
  1  MDPEFQEKTQ SLFANAFGYP ATHTIQAPGR VNLIGEHTDY NDGFVLPCAI
         galK←        →p120
 51  DYQTVIPGRV PVWKEATTTL FCASDAKAYD TEVHNVWATH ACVPTDPNPQ
101  EVVLVNVTEN FNMWKNDMVE QMHEDIISLW DQSLKPCVKL TPLCVSLKCT
151  DLKNDTNTNS SSGRMIMEKG EIKNCSFNIS TSIRGKVQKE YAFFYKLDII
                              p120←
201  PIDNDTTSYT LTSCNTSVIT QAS*
```

Construction of galK-p120 env(C) fusion:

A PvuII-HindIII filled-in fragment, comprising the end of p120 env and the beginning of p41 env was inserted at the StuI site of pOTSKF33 for in-frame fusion to the first 56 codons of galK. The resulting plasmid was digested with BglII (partial restriction) and ligated to a linker containing translation stops in all three frames (5′ CTA.GTT.AAC.TAG 3′). This plasmid expresses the carboxy-terminal (C) half of p120 env from codons number 258-437. The Ratner coordinates for this segment are: PvuII (6662) and BglII (7198).

The amino acid sequence of the galK-p120 env(C) fusion follows:

Length: 243 amino acid residues

```
  1  MDPEFQEKTQ SLFANAFGYP ATHTIQAPGR VNLIGEHTDY NDGFVLPCAI
         galK←        →p120
 51  DYQTVIPGRL NQSVEINCTR PNNNTRKSIR IQRGPGRAFV TIGKIGNMRQ
101  AHCNISRAKW NNTLKQIDSK LREQFGNNKT IIFKQSSGGD PEIVTHSFNC
151  GGEFFYCNST QLFNSTWFNS TWSTKGSNNT EGSDTITLPC RIKQIINMWQ
                                       p120←
201  EVGKAMYAPP ISGQIRCSSN ITGLLLTRDG GNSNNESEIL VN*
```

Expression of the galK-HIV chimeric antigens:

The various pOTSKF33 derivatives containing the galK-HIV antigen fusions were introduced in the E. coli AR120 strain and induced by treatment with nalidixic acid as described by Mott, et al., Proc. Natl. Acad. Sci. USA 82:88(1985). These expression vectors can also be induced by temperature shift (32°C → 42°C) in E. coli strains lysogenic for a defective and thermo-inducible prophage, e.g., N5151, Young, et al., supra.

## EXAMPLE 3

Purification of the galK-HIV chimeric antigens:

p55 gag

A 100 gm galK-p55 gag induced E. coli cell pellet was resuspended in 500 ml p55 lysis buffer (50mM Tris-HCl, pH 8.5, 5mM EDTA, 1mM DTT, 5%(v/v) glycerol and 0.1% thiodiglycol) containing 0.5 mg/ml lysozyme and 0.1 mg/ml each DNase and RNase. The mixture was incubated at room temperature for 15 minutes with constant mixing. All remaining steps were performed at 4°C.

The mixture was homogenized by passing 2-3 times through a Menton-Gaulin pressure homogenizer at 4500 psi. The homogenate was centrifuged at 10,000 x g for 30 minutes. The resulting pellet was resuspended in 500 ml p55 lysis buffer containing 0.2% sodium deoxycholate (NaDOC) and 1% Triton X-100 (v/v). This mixture was stirred for 15 minutes then centrifuged at 10,000 x g for 30 minutes.

The resulting pellet was resuspended in 500 ml p55 lysis buffer containing 1% Triton X-100 (v/v) and 0.5M KCl and stirred for 15 minutes before centrifuging at 10,000 x g for 30 minutes.

The resulting pellet was resuspended in 250 ml p55 lysis buffer containing 8M urea and allowed to stir for 30 minutes. The final centrifugation (10,000 x g for 30 minutes) results in the solubilized product being retained in the supernatant.

Chromatography on Superose 12 (Pharmacia Fine Chemicals, Uppsala, Sweden) in the presence of 2M guanidinium chloride yields a preparation of greater than 90% purity as determined by gel electrophoresis and staining with Coomassie Brilliant Blue. The yield of homogeneous material is approximately 150 mg/100 gm frozen E. coli pellet. The p55 gag fusion protein remains soluble when exchanged with phosphate buffered saline (PBS) at approximately 1-3 mg/ml.

## p41 env

The p41 env fusion protein was purified from galK-p41 env induced cells as described above for the p55 gag fusion protein with the exception that in the first centrifugation step, the mixture was centrifuged at low speed (1000 x g for 20 minutes), the supernatant carefully removed and centrifuged again at 27,000 x g for 20 minutes.

Chromatography on Superose-12 in 2M guanidinium chloride indicates that the env fusion protein behaves as a high molecular weight aggregate under these chromatography conditions. Mono S (Pharmacia Fine Chemicals, Uppsala, Sweden) chromatography in the presence of 8M urea gives a nonbound fraction of approximately 90% purity. A yield of approximately 300 mg/100 gm frozen E. coli pellet can be achieved. The p41 env fusion protein is not soluble when exchanged directly into PBS.

## p24 gag

Both of the extraction/purification procedures described above yield the p24 gag fusion protein at a purity of approximately 70%. Yield of the p24 gag fusion protein is approximately 300 mg/100 gm frozen induced E. coli pellet.

As the p120 env (N) and (C), and RTendo fusion proteins are also found in the insoluble fraction of the crude bacterial extract, it is expected that they can be purified using substantially the same procedure.

## EXAMPLE 4

Construction of the galK translation fusion vector, pOTSKF66:

pOTSKF66 derives from the pASI expression vector (Rosenberg, et al., supra) and comprises a large portion of the E. coli galK gene (253 codons) flanked by a polylinker with restriction sites for fusion in any of the three translation frames, stop codons for each phase and some additional cloning sites.

This plasmid was constructed by inserting a 761 bp BamHI-FspI fragment from pASK between the BamHI site and the XmaI-filled site of pOTSKF33. pOTSKF66 derivatives are introduced in the same E. coli strains and induced by the same treatments (nalidixic acid, heat) as the pOTSKF33 derivatives. Gene segments fused in frame to the 253 codons of galK in pOTSKF66 are expressed at high levels as observed with the pOTSKF33 fusion vector.

## EXAMPLE 5

Construction of the yeast galK translation fusion vector, pCD192-SKF33:

pCD192:

pCD192 derives from pYSK105 (Gorman et al., Gene 48:13(1986)). It was constructed in two steps:

Step 1: pYSK105 was digested with the NruI and PvuII restriction endonucleases and self-ligated with the T4 DNA ligase to delete the 1094 bp NruI-PvuII fragment. This treatment eliminated the unique PvuII site of the vector and yielded pYSK105-DNP.

Step 2: The 1.2 kilobase (kb) EcoRI fragment of pYSK105 carrying the galK coding sequence was excised and the EcoRI ends filled-in by treatment with DNA polymerase I (Klenow). The blunt ends were ligated to a PvuII linker with the sequence 5' CCAGCTGG 3'. The resulting plasmid was called pCD192 and will allow CUP1-directed expression of any gene (ATG initiation codon included) inserted at the unique PvuII site.

pCD192-SKF33:

pCD192-SKF33 derives from the pCD192 yeast expression vector, as described. pCD192-SKF33 is a pBR322-based shuttle vector that comprises a partial 2 micron origin of replication, the trp1 selective marker, the copper-regulatable CUP1 promoter followed by a portion of the E. coli galK gene (56 codons) flanked by a polylinker with restriction sites for fusion in any of the three translation frames, stop codons for each phase and some additional cloning sites. This vector was constructed in two steps:

Step 1: deletion of the BamHI, SmaI/XmaI, SphI and SalI sites of pCD192:

pCD192 was digested with the BamHI and SalI restriction endonucleases, treated with DNA polymerase I (Klenow) to create blunt ends and self-ligated with T4 DNA ligase. This treatment deletes a 286 bp fragment position upstream from the CUP1 promoter in pCD192 thereby removing the BamHI, SmaI/XmaI, SphI and SalI sites and yields plasmid pCD192-DBS.

Step 2: insertion of the partial galK expression unit from pOTSKF33 into pCD192-DBS:

pCD192-DBS was digested with the PvuII restriction endonuclease which cuts immediately downstream from the CUP1 promoter. It was ligated to a 313 bp NdeI-XhoI filled-in fragment isolated and purified from pOTSKF33. This fragment encompassed the ATG initiation codon and the 56 amino-terminal codons of E. coli galK flanked by restriction sites for fusion in any of the three translation frames and stop codons for each phase. This yields the final vector pCD192-SKF33.

pCD192-SKF33 was shown to be useful in increasing the level of expression of gene segments which are poorly or not expressed when unfused, as has been shown for the same translation fusion system in E. coli (pOTSKF33, Example 1).

## EXAMPLE 6

Construction and expression of galK-βglobin protein fusion in yeast:

A 1381 bp NcoI-XbaI filled-in fragment containing the full length coding sequence of the human βglobin chain preceded by the recognition sequence for the factor Xa protease (Ile-Glu-Gly-Arg), was isolated from the pJK06 plasmid (O'Donnell, Ph.D. thesis, Temple University, 1987) and inserted at the SmaI site of pCD192-SKF33 for in-frame fusion to the first 56 codons of galK.

This pCD192-SKB33 derivative containing the galK-βglobin fusion was introduced in the yeast Saccharomyces cerevisiae BR10 strain by the lithium chloride (LiCl) method (Ito et al., J. Bacteriol. 153:163(1983)). The CUP1 promoter was induced by addition of CuSo4 as folows: BR10 cells containing the pCD192-SKF33 derivative with the galK-βglobin fusion were grown to log phase at 30°C in minimum medium with glucose and without tryptophane. CuSo4 was then added to a final concentration of 0.1 mM. After an additional hour of aeration at 30°C, the cells were collected by centrifugation and washed once with water. The extracts were prepared by lysing the cells with glass beads and were analyzed by SDS-PAGE and immunoblot with a βglobin-specific antibody. It was observed that the βglobin gene product was expressed at a much higher level when fused to the 56 codons of galK than when unfused.

The above description and examples fully disclose the invention including preferred embodiments thereof. Modifications of the methods described that are obvious to those of ordinary skill in molecular genetics and related sciences are intended to be within the scope of the following claims.

**Claims**

1. An immunodiagnostic device for assaying for the presence of antibodies to a particular antigen which comprises:
   a) a solid support;
   b) an antibody bound to the solid support;
   c) a chimeric antigen having a first and a second antigenic region, the first antigenic region being reactive with the bound antibody but not being cross-reactive with antibodies recognizing the second antigenic region.

2. The device of claim 1 wherein the second antigenic region is representative of a pathogenic microorganism, protein, virus or cell.

3. The device of claim 2 wherein the first antigenic region is representative of a protein or protein segment which will not recognize binding sites in an animal body fluid.

4. The device of claim 3 wherein the second antigenic region is an antigen derived from a protein associated with specific cancers, genetic abnormalities, bacterial, fungal, viral or parasitic diseases.

5. A process for preparing chimeric HIV-antigens which comprises fusing a portion of the galK gene to an HIV gene segment and introducing the resulting fused gene segment into an organism and culturing the organism such that the chimeric HIV-antigen is expressed.

6. The process of claim 5 in which the chimeric HIV-antigen accumulates at high levels and is stable when expressed in E. coli.

7. The process of claim 5 in which the chimeric antigen is encoded by a gene coding sequence for galK fused to p24 gag coding sequences of HIV.

8. The process of claim 5 in which the chimeric antigen is encoded by a gene coding sequences for galK fused to p55 gag coding sequence of HIV.

9. The process of claim 5 in which the chimeric antigen is encoded by a gene coding sequence for galK fused to reverse transcriptase-endonuclease (RTendo) coding sequences of HIV.

10. The process of claim 5 in which the chimeric antigen is encoded by a gene coding sequence for galK fused to p41 env coding sequences of HIV.

11. The process of claim 5 in which the chimeric antigen is encoded by a gene coding sequence for galK fused to p120 env amino terminal half (N) coding sequences of HIV.

12. The process of claim 5 in which the chimeric antigen is encoded by a gene coding sequence for galK fused to p120 env carboxy terminal half (C) coding sequences of HIV.

13. A method for detection of anti-HIB antibodies in a sample comprising contacting a sample with a galK-HIV chimeric antigen bound to a solid support such that only those samples containing HIV-specific immunoglobulins bind to the HIV-chimeric antigen.

14. The method of claim 13 wherein the chimeric antigen is attached to a solid support by means of the galK region that all HIV epitopes present on the molecule are available for recognition by the sample.

15. A vector comprising a promoter and a portion of the galK gene flanked by a coding sequence for an antigen not cross-reactive with galK.

16. The galK translation fusion vector of claim 15 known as galK-p24 gag, galK-p55 gag, galK-RTendo, galK-p41 env, galK-p120 env(N) or galK-p120 env(C).

17. An E. coli transformed with a galK translation fusion vector as described in claim 16.

18. A chimeric HIV antigen which comprises a HIV antigenic region and a second antigenic region, the second antigenic region not being cross-reactive with antibodies recognizing HIV antigenic region.

19. A chimeric HIV antigen according to claim 18 in which the second antigenic region comprises a portion of the E. coli galK gene.

20. A chimeric HIV antigen according to claim 18 which is encoded by a gene coding sequence for galK fused to p24 gag coding sequences of HIV.

21. A chimeric HIV antigen according to claim 18 which is encoded by a gene coding sequence for galK fused to p55 gag coding sequences of HIV.

22. A chimeric HIV antigen according to claim 18 which is encoded by a gene coding sequence for galK fused to reverse transcriptase-endonuclease (RTendo) coding sequences of HIV.

23. A chimeric HIV antigen according to claim 18 which is encoded by a gene coding sequence for galK fused to p41 env coding sequences of HIV.

24. A chimeric HIV antigen according to claim 18 which is encoded by a gene coding sequence for galK fused to p120 env amino terminal half (N) coding sequences of HIV.

25. A chimeric HIV antigen according to claim 18 which is encoded by a gene coding sequence for galK fused to p120 env carboxy terminal half (C) coding sequences of HIV.

26. A diagnostic kit comprising a device as claimed in any one of claims 1 to 4.

## Claims for the following contracting States: ES, GR

1. An immunodiagnostic device for assaying for the presence of antibodies to a particular antigen which comprises:

a) a solid support;

b) an antibody bound to the solid support;

c) a chimeric antigen having a first and a second antigenic region, the first antigenic region being reactive with the bound antibody but not being cross-reactive with antibodies recognizing the second antigenic region.

2. The device of claim 1 wherein the second antigenic region is representative of a pathogenic microorganism, protein, virus or cell.

3. The device of claim 2 wherein the first antigenic region is representative of a protein or protein segment which will not recognize binding sites in an animal body fluid.

4. The device of claim 3 wherein the second antigenic region is an antigen derived from a protein associated with specific cancers, genetic abnormalities, bacterial, fungal, viral or parasitic diseases.

5. A process for preparing chimeric HIV-antigens which comprises fusing a portionof the galK gene to an HIV gene segment and introducing the resulting fused gene segment into an organism and culturing the organism such that the chimeric HIV-antigen is expressed.

6. The process of claim 5 in which the chimeric HIV-antigen accumulates at high levels and is stable when expressed in E. coli.

7.The process of claim 5 in which the chimeric antigen is encoded by a gene coding sequence for galK fused to p24 gag coding sequences of HIV.

8. The process of claim 5 in which the chimeric antigen is encoded by a gene coding sequence for galK fused to p55 gag coding sequences of HIV.

9. The process of claim 5 in which the chimeric antigen is encoded by a gene coding sequence for galK fused to reverse transcriptase-endonuclease (RTendo) coding sequences of HIV.

10. The process of claim 5 in which the chimeric antigen is encoded by a gene coding sequence for galK fused to p41 env coding sequences of HIV.

11. The process of claim 5 in which the chimeric antigen is encoded by a gene coding sequence for galK fused to p120 env amino terminal half (N) coding sequences of HIV.

12. The process of claim 5 in which the chimeric antigen comprises a gene coding sequence for galK fused to p120 env carboxy terminal half (C) coding sequences of HIV.

13. A method for detection of anti-HIV antibodies in a sample comprising contacting a sample with a galK-HIV chimeric antigen bound to a solid support such that only those samples containing HIV-specific immunoglobulins bind to the HIV-chimeric antigen.

14. The method of claim 13 wherein the chimeric antigen is attached to a solid support by means of the

**0 293 184**

galK region that all HIV epitopes present on the molecule are available for recognition by the sample.

12